# EUROPEAN PATENT APPLICATION

(11) **EP 3 736 262 A1**
(43) Date of publication of application: **11.11.2020**
(21) Application number: 19173248.6
(22) Date of filing: 08.05.2019
(51) Int. Cl.: C07C 231/24, C07C 233/09

(54) **PROCESS FOR THE PURIFICATION OF A SOLUTION COMPRISING ACRYLAMIDE MONOMER**

(71) Applicant: BASF SE, 67056 Ludwigshafen am Rhein (DE)
(72) Inventor: GHISLIERI, Diego, 67056 Ludwigshafen (DE); ZIMMERMANN, Tobias Joachim, 67056 Ludwigshafen (DE); VARGAS SCHMITZ, Juergen Jose, 67056 Ludwigshafen (DE); OEDMAN, Peter, Saint Joseph, MO 64507 (US); LANG, Hans-Juergen, 67056 Ludwigshafen (DE)
(74) Representative: BASF IP Association

(57) **Abstract**

The presently claimed invention is directed to a method for the purification of a solution comprising acrylamide.

## Description

### Field of invention

The presently claimed invention is directed to a method for the purification of a solution comprising acrylamide.

### Background of Invention

Acrylamide is used as a raw material for polyacrylamides which are used for many applications such as water treating agents, aggregating agents, thickening agents, oil recovery agents, friction reducers in hydraulic fracturing, paper power enhancing agents in the papermaking industry and thickening agents for papermaking. The most common method for the preparation of the acrylamide monomer is hydrolysis of the acrylonitrile using a microbial cell having the nitrile hydratase activity. The method of using a microbial cell having the nitrile hydratase activity is remarkably effective for the industrial production of the acrylamide monomer, because the reaction conditions are mild with almost no by-product formation.

The aqueous acrylamide solution prepared by the catalytic hydration process tends to undergo coloration or to become turbid immediately after the preparation thereof, or with the passage of time, due to trace amounts of impurities, such as substances eluted from the biocatalyst used, by-products, and the like. The aqueous acrylamide solution prepared by the biocatalytic hydration process contains impurities like surface-active agents originating either from the ruptured cell or by-product of the fermentation process, such as defoamers etc. These surface-active agents cause a strong foaming behaviour which impacts the throughput of the downstream polymer process. Thus, it is desirable to reduce the amount of surface-active agents present in the acrylamide solution.

The crude aqueous acrylamide thus produced is purified by passing the aqueous solution through a column packed with activated carbon, particularly granular activated carbon. However, the acrylamide monomer can easily be polymerized by light, heat, and when brought into contact with a surface of iron. It is also observed that acrylamide is very apt to be polymerized around activated carbon, thus causing obstruction of the column. As a result, the advantages of the granular activated carbon cannot be fully utilized.

US 3,923,741 discloses incorporating cupric ion in the activated carbon to avoid the polymerization of the acrylamide while purification. While purification, the cupric ion tends to be extracted into the aqueous solution, resulting in not only reduction of cupric ions adsorbed on the activated carbon to readily cause polymerization of acrylamide around the activated carbon, but also incorporation of cupric ions that were not formerly present in the crude aqueous solution in the purified solution.

EP 0 182 578 A1 discloses a method, whereby the activated carbon is contacted with water having dissolved oxygen until the dissolved oxygen concentration in water after the contact increases to at least 0.5 ppm. Then the acrylamide solution is passed through the activated carbon to purify the crude acrylamide. However, the drawback of this process resides in maintaining the concentration of the solution comprising acrylamide at 20 wt.-% and the temperature of the activated carbon column at ≤ 10 °C.

EP 1 167 345 A1 discloses a method for eliminating impurities contained in a solution comprising acrylamide monomer by bringing the solution into contact with active carbon under acidic pH in the range of ≥ 3.5 to ≤ 6.5. The preferred pH of the reaction for conversion of acrylonitrile to acrylamide is ≥ 6 to ≤ 10. However, the pH is then adjusted to a level of ≥ 3.5 to ≤ 6.5 for the purification. Further, the activated carbon is filtered off to get the purified acrylamide solution.

Thus, there is a constant need for devising optimum conditions for the purification of the crude acrylamide solution obtained by hydrolysis of acrylonitrile, in particular the hydrolysis performed using a microbial cell having nitrile hydratase activity.

Thus, the object of the presently claimed invention is to provide a process for efficiently removing impurities, in particular impurities which cause foaming, which are present in the solution comprising acrylamide.

### Summary of Invention

Surprisingly, it was found that the impurities which are present in the acrylamide solution, especially the impurities such as surface-active agents, can be effectively removed or at least reduced at ambient temperature and at a pH ≥ 6.0 to ≤ 9.0 without causing unwanted polymerization of the acrylamide monomer by using a fixed-bed reactor comprising activated carbon that is treated with oxygen and at least one stabilizer.

Thus, in one aspect, the presently claimed invention is directed to a method for the purification of a solution comprising acrylamide comprising at least the steps of:
a) providing a fixed-bed reactor comprising activated carbon;
d) treating the activated carbon with oxygen and at least one stabilizer; and
f) passing the solution comprising acrylamide through the fixed-bed reactor to obtain a purified solution comprising acrylamide;
wherein the pH of the solution comprising acrylamide is in the range of ≥ 6.0 to ≤ 9.

### Detailed deception of invention

Before the present compositions and formulations of the presently claimed invention are described, it is to be understood that this invention is not limited to particular compositions and formulations described, since such compositions and formulation may, of course, vary. It is also to be understood that the terminology used herein is not intended to be limiting, since the scope of the presently claimed invention will be limited only by the appended claims.

If hereinafter a group is defined to comprise at least a certain number of embodiments, this is meant to also encompass a group which preferably consists of these embodiments only. Furthermore, the terms 'first', 'second', 'third' or 'a', 'b', 'c', etc. and the like in the description and in the claims, are used for distinguishing between similar elements and not necessarily for describing a sequential or chronological order. It is to be understood that the terms so used are interchangeable under appropriate circumstances and that the embodiments of the presently claimed invention described herein are capable of operation in other sequences than described or illustrated herein. In case the terms 'first', 'second', 'third' or '(A)', '(B)' and '(C)' or '(a)', '(b)', '(c)', '(d)', 'i', 'ii' etc. relate to steps of a method or use or assay there is no time or time interval coherence between the steps, that is, the steps may be carried out simultaneously or there may be time intervals of seconds, minutes, hours, days, weeks, months or even years between such steps, unless otherwise indicated in the application as set forth herein above or below.

Furthermore, the ranges defined throughout the specification include the end values as well i.e. a range of 1 to 10 implies that both 1 and 10 are included in the range. For the avoidance of doubt, applicant shall be entitled to any equivalents according to applicable law.

In the following passages, different aspects of the presently claimed invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to 'one embodiment' or 'an embodiment' means that a particular feature, structure or characteristic described in connection with the embodiment is included in at least one embodiment of the presently claimed invention. Thus, appearances of the phrases 'in one embodiment' or 'in an embodiment' in various places throughout this specification are not necessarily all referring to the same embodiment, but may refer to the same embodiment. Further, as used in the following, the terms "preferably", "more preferably", "even more preferably", "most preferably" and "in particular" or similar terms are used in conjunction with optional features, without restricting alternative possibilities. Thus, features introduced by these terms are optional features and are not intended to restrict the scope of the claims in any way.

Furthermore, the particular features, structures or characteristics may be combined in any suitable manner, as would be apparent to a person skilled in the art from this disclosure, in one or more embodiments. Furthermore, while some embodiments described herein include some, but not other features included in other embodiments, combinations of features of different embodiments are meant to be within the scope of the presently claimed invention, and form different embodiments, as would be understood by those in the art. For example, in the appended claims, any of the claimed embodiments can be used in any combination.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

In a first embodiment, the presently claimed invention is directed to a process for the purification of a solution comprising acrylamide comprising at least the steps of:
a) providing a fixed-bed reactor comprising activated carbon;
d) treating the activated carbon with oxygen and at least one stabilizer; and
f) passing the solution comprising acrylamide through the fixed-bed reactor to obtain a purified solution comprising acrylamide;
wherein the pH of the solution comprising acrylamide is in the range of ≥ 6.0 to ≤ 9.

More preferably, the presently claimed invention is directed to a process for the purification of a solution comprising acrylamide comprising at least the steps of:
a) providing a fixed-bed reactor comprising activated carbon;
d) treating the activated carbon with oxygen and at least one stabilizer; and
f) passing the solution comprising acrylamide through the fixed-bed reactor to obtain a purified solution comprising acrylamide;
wherein the pH of the solution comprising acrylamide is in the range of ≥ 6.0 to ≤ 8.5, more preferably in the range of ≥ 6.5 to ≤ 8.5, even more preferably in the range of ≥ 7.0 to ≤ 8.0 and most preferably in the range of ≥ 6.5 to ≤ 8.0.

In another preferred embodiment, the presently claimed invention is directed to a process for the purification of a solution comprising acrylamide comprising at least the steps of:
a) providing a fixed-bed reactor comprising activated carbon;
d) treating the activated carbon with oxygen and at least one stabilizer; and
f) passing the solution comprising acrylamide through the fixed-bed reactor to obtain a purified solution comprising acrylamide;
wherein the pH of the solution comprising acrylamide is in the range of ≥ 7.0 to ≤ 8.0; and in each case the acrylamide is present in the solution comprising acrylamide in a concentration in the range of ≥ 30.0 wt.-% to ≤ 60.0 wt.-%, based on the total weight of the solution.

In another preferred embodiment, the process for purification of a solution comprising acrylamide comprises at least the steps of:
a) providing a fixed-bed reactor comprising activated carbon;
b) washing the fixed-bed reactor comprising activated carbon with water;
c) dumping the water using air;
d) treating the activated carbon with oxygen and at least one stabilizer;
e) loading of the acrylamide solution to the fixed-bed reactor comprising activated carbon; and
f) passing the solution comprising acrylamide through the fixed-bed reactor to obtain a purified solution comprising acrylamide.

### Solution comprising acrylamide

In another preferred embodiment, the acrylamide is obtained by the hydrolysis of acrylonitrile by a nitrile hydratase. The nitrile hydratase is an enzyme produced by different microorganisms.

In context with the present invention, microorganisms naturally encoding nitrile hydratase, comprise species belonging to a genus selected from the group consisting of *Rhodococcus, Aspergillus, Acidovorax, Agrobacterium, Bacillus, Bradyrhizobium, Burkholderia, Escherichia, Geobacillus, Klebsiella, Mesorhizobium, Moraxella, Pantoea, Pseudomonas, Rhizobium, Rhodopseudomonas, Serratia, Amycolatopsis, Arthrobacter, Brevibacterium, Corynebacterium, Microbacterium, Micrococcus, Nocardia, Pseudonocardia, Trichoderma, Myrothecium, Aureobasidium, Candida, Cryptococcus, Debaryomyces, Geotrichum, Hanseniaspora, Kluyveromyces, Pichia, Rhodotorula, Comomonas, and Pyrococcus.* In preferred embodiments of the invention the microorganism is selected from bacteria of the genus *Rhodococcus, Pseudomonas, Escherichia* and *Geobacillus.*

Preferred microorganisms to be employed in context with any one of the methods of the present invention comprise representatives of the genus *Rhodococcus, e.g., Rhodococcus rhodochrous (e.g., NC*/*MB 41164, J1*/*FERM-BP 1478, M33 or M8), Rhodococcus pyridinovorans, Rhodococcus erythropolis, Rhodococcus equi, Rhodococcus ruber, or Rhodococcus opacus.* Further, species suitable as microorganism to be employed in context with any one of the methods of the present invention are, e.g., *Aspergillus niger, Acidovorax avenae, Acidovorax facilis, Agrobacterium tumefaciens, Agrobacterium radiobacter, Bacillus subtilis, Bacillus pallidus, Bacillus smithii, Bacillus sp BR449, Bradyrhizobium oligotrophicum, Bradyrhizobium diazoefficiens, Bradyrhizobium japonicum, Burkholderia cenocepacia, Burkholderia gladioli, Escherichia coli, Geobacillus sp. RAPc8, Klebsiella oxytoca, Klebsiella pneumonia, Klebsiella variicola, Mesorhizobium ciceri, Mesorhizobium opportunistum, Mesorhizobium* sp F28, *Moraxella, Pantoea endophytica, Pantoea agglomerans, Pseudomonas chlororaphis, Pseudomonas putida, Rhizobium, Rhodopseudomonas palustris, Serratia liquefaciens, Serratia marcescens, Amycolatopsis, Arthrobacter, Brevibacterium sp CH1, Brevibacterium sp CH2, Brevibacterium sp R312, Brevibacterium imperiale, Brevibacterium casei, Corynebacterium nitrilophilus, Corynebacterium pseudodiphteriticum, Corynebacterium glutamicum, Corynebacterium hoffmanii, Microbacterium imperiale, Microbacterium smegmatis, Micrococcus luteus, Nocardia globerula, Nocardia rhodochrous, Nocardia sp 163, Pseudonocardia thermophila, Trichoderma, Myrothecium verrucaria, Aureobasidium pullulans, Candida famata, Candida guilliermondii, Candida tropicalis, Cryptococcus flavus, Cryptococcus sp* UFMG- Y28, *Debaryomyces hanseii, Geotrichum candidum, Geotrichum* sp JR1, *Hanseniaspora, Kluyveromyces thermotolerans, Pichia kluyveri, Rhodotorula glutinis, Comomonas testosteroni, Pyrococcus abyssi, Pyrococcus furiosus, or Pyrococcus horikoshii.*

According to one embodiment of any one of the methods of the present invention, the microorganisms belong to the species *Rhodococcus rhodochrous.* Particular examples for strains belonging to *Rhodococcus rhodochrous* which may be employed in context with any one of the methods described herein comprise NCIMB 41164, J1 (FERM-BP 1478), M33 and M8. Alternatively or in addition to *Rhodococcus rhodochrous,* the microorganism employed in any one of the methods described herein may be *Rhodococcus pyridinovorans.*

In context with the present invention, nitrile hydratase encoding microorganisms which are not naturally encoding nitrile hydratase may be genetically engineered microorganisms which naturally do not contain a gene encoding a nitrile hydratase but which have been manipulated such as to contain a polynucleotide encoding a nitrile hydratase (e.g., via transformation, transduction, transfection, conjugation, or other methods suitable to transfer or insert a polynucleotide into a cell as known in the art; cf. Sambrook and Russell 2001, Molecular Cloning: A Laboratory Manual, CSH Press, Cold Spring Harbor, NY, USA), thus enabling the microorganisms to produce and stably maintain the nitrile hydratase enzyme. For this purpose, it may further be required to insert additional polynucleotides which may be necessary to allow transcription and translation of the nitrile hydratase gene or mRNA, respectively. Such additional polynucleotides may comprise, *inter alia,* promoter sequences, polyT- or polyU-tails, or replication origins or other plasmid-control sequences. In this context, such genetically engineered microorganisms which naturally do not contain a gene encoding a nitrile hydratase, but which have been manipulated such as to contain a polynucleotide encoding a nitrile hydratase may be prokaryotic or eukaryotic microorganisms. Examples for such prokaryotic microorganisms include, e.g., representatives of the species *Escherichia coli.* Examples for such eukaryotic microorganisms include, e.g., yeast (e.g., *Saccharomyces cerevisiae*).

In the context of the present invention, the term "nitrile hydratase" generally means an enzyme which is capable of catalyzing the conversion (*i.e.* hydration) of acrylonitrile to acrylamide. Such an enzyme may be, e.g., the enzyme registered under IUBMB nomenclature as of September 30, 2014: EC 4.2.1.84; CAS-No. 2391-37-5. However, the term "nitrile hydratase" as used herein also encompasses modified or enhanced enzymes which are, e.g., capable of converting acrylonitrile to acrylamide more quickly, or which can be produced at a higher yield/time-ratio, or which are more stable, as long as they are capable to catalyze conversion (*i.e.* hydration) of acrylonitrile to acrylamide. Methods for determining the ability of a given microorganism or enzyme for catalyzing the conversion of acrylonitrile to acrylamide are known in the art. As an example, in context with the present invention, activity of a given microorganism or enzyme to act as a nitrile hydratase in the sense of the present invention may be determined as follows: First reacting 100 µl of a cell suspension, cell lysate, dissolved enzyme powder or any other preparation containing the supposed nitrile hydratase with 875 µl of an 50 mM potassium phosphate buffer and 25 µl of acrylonitrile at 25 °C on an Eppendorf tube shaker at 1,000 rpm for 10 minutes. After 10 minutes of reaction time, samples may be drawn and immediately quenched by adding the same volume of 1.4% hydrochloric acid. After mixing of the sample, cells may be removed by centrifugation for 1 minute at 10,000 rpm and the amount of acrylamide formed is determined by analyzing the clear supernatant by HPLC. For affirmation of an enzyme to be a nitrile hydratase in context with the present invention, the concentration of acrylamide shall be between 0.25 and 1.25 mmol/l - if necessary, the sample has to be diluted accordingly and the conversion has to be repeated. The enzyme activity may then be deduced from the concentration of acrylamide by dividing the acrylamide concentration derived from HPLC analysis by the reaction time, which has been 10 minutes and by multiplying this value with the dilution factor between HPLC sample and original sample. Activities >5 U/mg dry cell weight, preferably >25 U/mg dry cell weight, more preferably >50 U/mg dry cell weight, most preferably >100 U/mg dry cell weight indicate the presence of a functionally expressed nitrile hydratase and are considered as nitrile hydratase in context with the present invention.

Upon producing an amide compound by using the foregoing microorganisms, a fungus body or a processed product of a fungus body is generally used. The fungus body can be prepared by utilizing an ordinary method having been known in the fields of molecular biology, bioengineering and genetic engineering. For example, such a method can be exemplified that after the microorganism is planted in an ordinary liquid culture medium, such as an LB medium, an M9 medium and the like, it is grown at an appropriate culture temperature (which is generally from 20 °C to 50 °C, and 50 °C or higher is possible for thermophile), and then the microorganism is separated and recovered from the culture liquid by centrifugal separation.

The processed product of the microorganism fungus body in the invention denotes an extract and a trituration product of the microorganism fungus body, a post-separated product obtained by separating and purifying a nitrile hydratase active fraction of the extract and the trituration product, and a fixed product obtained by fixing the microorganism fungus body, or the extract, the trituration product or the post-separated product of the fungus body to an appropriate carrier, and these are included in the processed product of the fungus body of the invention as far as they has an activity as nitrile hydratase.

In another preferred embodiment, the acrylamide is present in the solution in a concentration in the range of ≥ 30.0 wt.-% to ≤ 60.0 wt.-%, based on the total weight of the solution. More preferably the concentration is in the range of ≥ 35.0 wt.-% to ≤ 55.0 wt.-%, most preferably the concentration is in the range of ≥ 40.0 wt.-% to ≤ 55.0 wt.-%, and in particular the concentration is in the range of ≥ 45.0 wt.-% to ≤ 55.0 wt.-%, based on the total weight of the solution.

In another preferred embodiment, the solution comprising acrylamide has a pH in the range of ≥ 6.0 to ≤ 9.0. More preferably, the pH is in the range of ≥ 6.5 to ≤ 8.5; even more preferably the pH is in the range of ≥ 6.6 to ≤ 8.5; most preferably the pH is in the range of ≥ 6.7 to ≤ 8; and in particular the pH is in the range of ≥ 6.8 to ≤ 7.5.

The inventively claimed process is used to purify a solution comprising acrylamide by removing impurities such as surface-active agents and proteins, to obtain a purified solution comprising acrylamide. The surface-active agents and the proteins are resulted from the microbial process used for the preparation of the acrylamide monomer by hydrolysing the acrylonitrile. The surface-active agents and the proteins are mixed with the solution comprising acrylamide monomer is liable to be formed when they are present in traces amount and the solution comprising acrylamide is liable to be clouded when the amount of the surface-active agents and the proteins are large.

In another preferred embodiment, the solution comprising acrylamide comprises at least one surface-active agent having a surface tension ≤ 60 mN/m.

In another preferred embodiment, the at least one surface-active agent present in the solution comprising acrylamide is a non-ionic surfactant.

Non-ionic surfactants have covalently bonded oxygen-containing hydrophilic groups, which are bonded to hydrophobic parent structures. The water-solubility of the oxygen groups is the result of hydrogen bonding. Hydrogen bonding decreases with increasing temperature, and the water solubility of non-ionic surfactants therefore decreases with increasing temperature.

In another preferred embodiment, the at least one surface active agent is present in the solution in an amount in the range of ≥ 1 ppm to ≤ 1000 ppm, based on the total weight of the solution. More preferably, the at least one surface active agent is present in the solution in an amount in the range of ≥ 3 ppm to ≤ 100 ppm, based on the total weight of the solution. Even more preferably, the at least one surface active agent is present in the solution in an amount in the range of ≥ 5 ppm to ≤ 1000 ppm, based on the total weight of the solution and most preferably, the at least one surface active agent is present in the solution in an amount in the range of ≥ 10 ppm to ≤ 50 ppm, based on the total weight of the solution.

In another preferred embodiment, the at least one surface active agent is present in the purified solution in an amount in the range of ≥ 0.001 ppm to ≤ 3 ppm , based on the total weight of the solution. More preferably, the at least one surface active agent is present in the purified solution in an amount in the range of ≥ 0.001 ppm to ≤ 1ppm, based on the total weight of the solution. Most preferably, the at least one surface active agent is present in the purified solution in an amount in the range of ≥ 0.01 ppm to ≤ 1ppm, based on the total weight of the solution.
In another preferred embodiment, the at least one surface active agent is selected from the group consisting of polyalkylene glycol, alkyl polyalkylene glycol, alkyl polyalkoxyester, fatty acid polyglycol esters, polysiloxane glycol copolymers, silicone compounds and emulsions, oxalkylated compounds, mineral oil/synthetic blends, glycol/ester blends.

In another preferred embodiment, the solution comprises proteins in an amount in the range of ≥ 10 mg/L to ≤ 2 g/L, based on the total weight of the solution.

In another preferred embodiment, the purified solution comprises proteins in an amount in the range of ≥ 0.1 mg/L to ≤ 8 mg/L, based on the total weight of the solution.

### Fixed-bed reactor and activated carbon

The inventively claimed process is carried out in a fixed-bed reactor, preferably in a continuous fixed-bed reactor packed with activated carbon.

A fixed bed reactor is a cylindrical tube filled with the activated carbon with a solution comprising acrylamide monomer is flowing through the bed. The activated carbon bed may have multiple configuration including: one large bed, several horizontal beds, several parallel packed tubes, multiple beds in their own shells.

In another preferred embodiment, the activated carbon that is used is present in powdered, granular, pellet or cylindrical shape.

The activated carbons are porous solids with very high surface areas. They can be derived from a variety of sources including coal, wood, coconut husk, nutshells, and peat. Activated carbon can be produced from these materials using physical activation involving heating under a controlled atmosphere or chemical activation using strong acids, bases, or oxidants. The activation processes produce a porous structure with high surface areas that give activated carbon high capacities for impurity removal. Activation processes can be modified to control the acidity of the surface.

In another embodiment, the activation processes involve subjecting a carbon source, such as resin wastes, coal, coal coke, petroleum coke, lignites, polymeric materials, and lignocellulosic materials including pulp and paper, residues from pulp production, wood (like wood chips, sawdust, and wood flour), nut shell (like almond shell and coconut shell), kernel, and fruit pits (like olive and cherry stones) to a thermal process (e.g., with an oxidizing gas) or a chemical process (e.g., with phosphoric acid or metal salts, such as zinc chloride).

In another preferred embodiment, the activated carbon is selected from the group consisting of steam activated carbon, acid activated carbon or activated using at least one chemical activating agents selected from the group consisting of alkali metal hydroxides, alkali metal carbonates, alkali metal sulfide, alkali metal sulfates, alkaline earth metal carbonates, alkaline earth metal chlorides, alkaline earth metal sulfates and alkaline earth metal phosphates.

In another preferred embodiment, the activated carbon has a particle size in the range of ≥ 50 nm to ≤ 8 mm, determined according to sedigraph measurements. More preferably, the particle diameter of activated carbon ranges from ≥ 0.3 mm to ≤15 mm, determined according to sedigraph measurements, and even more preferably, it ranges from ≥ 0.8 mm to ≤ 6 mm, determined according to sedigraph measurements.

In another embodiment, the activated carbon products exhibit a surface area of ≥ 300 to ≤ 2500 m²/g, as measured by the nitrogen adsorption based Brunauer-Emmett-Teller ("BET") method. More preferably, the activated carbon has a surface area in the range of ≥ 300 to ≤ 1600 m²/g, determined according to BET surface area analysis.

In another embodiment, the activated carbon has the porosity is in the range of ≥ 0.05 to ≤ 500 nm, determined according to Hg intrusion porosimetry.

In another embodiment, the activated carbon has a porosity of at least about 0.25 cc/g in pores diameter of ≥ 0.1 nm to ≤ 50 nm. More preferably, the activated carbon has a porosity of at least about 0.4 cc/g in pores diameter of ≥ 0.1 nm to ≤ 50 nm.

In another embodiment, the activated carbon has a relatively high percentage of its pores as mesoporous and relatively low percentages of its pores as microporous.

In another preferred embodiment, the microporosity refers to pores having a size (average cross-section) of less than 2 nm, mesoporosity refers to pores having a size from ≥ 2 nm to ≤ 50 nm, while macroporosity refers to pores having a size ≥ 50 nm. A major amount refers to 50% or more while a minor amount refers to less than 50%.

In another embodiment, the level of porosity in the activated carbon is less than 50% microporosity, from 50% to about 100% mesoporosity and/or macroporosity. More preferably, the levels of porosity in the activated carbon are less than about 40% microporosity, from about 60% to about 99% mesoporosity and/or macroporosity. Most preferably, the levels of porosity in the activated carbon are less than about 35% microporosity, from about 65% to about 95% mesoporosity and/or macroporosity.

In one embodiment, in the mesoporosity and/or macroporosity fraction, the levels of porosity are from about ≥ 0% to about ≤ 100% mesoporosity and from about ≥ 100% to about ≤ 0% macroporosity. More preferably, in the mesoporosity and/or macroporosity fraction, the level of mesoporosity is greater than the level of macroporosity and most preferably, in the mesoporosity and/or macroporosity fraction, the levels of porosity are from ≥ 60% to ≤ 99% mesoporosity and from ≥ % to ≤ 40% macroporosity.

### Process steps

In another preferred embodiment, the process for purification of a solution comprising acrylamide comprises at least the steps of:
a) providing a fixed-bed reactor comprising activated carbon;
b) washing the fixed-bed reactor comprising activated carbon with water;
c) dumping the water using air;
d) treating the activated carbon with oxygen and at least one stabilizer;
e) loading of the acrylamide solution to the fixed-bed reactor comprising activated carbon; and
f) passing the solution comprising acrylamide through the fixed-bed reactor to obtain a purified solution comprising acrylamide.

In yet another embodiment, the process is preferably carried out in a batch mode, semi-batch mode or continuous mode. The process is preferably carried out in the continuous mode.

In another embodiment, the batch mode is a closed process in which, a fixed amount of the material is treated each time it operates.

In another embodiment, the semi-batch mode is an open process in which materials enter the process during its operation, but none leaves.

In another embodiment, the continuous mode is a closed process in which the materials enter and leave the system without interruption.

In another preferred embodiment, in step d) the activated carbon is treated by using a water solution that is saturated with oxygen and contains the at least one stabilizer; or the activated carbon is treated by using humid air that is saturated with oxygen and a water solution that contains the at least stabilizer. More preferably, in step d) the activated carbon is treated by using a water solution that is saturated with oxygen and contains the at least one stabilizer; or the activated carbon is treated by using water vapour that is saturated with oxygen and a water solution that contains the at least stabilizer.

The at least one stabilizer is capable of acrylamide against oxidative degradation. The at least one stabilizer is preferably selected from the group consisting of phenoxazine, phenothiazine and hydroquinone-type stabilizers. Phenoxazines include 10H-phenoxazine, which is the unsubstituted phenoxazine, as well as substituted phenoxazines in which the aryl hydrogens may be independently substituted by linear or branched, aliphatic or aromatic C₁-C₁₈ moiety. Phenothiazines include 10H-phenothiazine, which is unsubstituted phenothiazine, as well as substituted phenothiazines in which the aryl hydrogens may be independently substituted by linear or branched, aliphatic or aromatic C₁-C₁₈ moiety.

Hydroquinone-type stabilizing compounds are capable of stabilizing other compounds against oxidative degradation. Hydroquinones are the para isomers of dihydroxybenzenes and include unsubstituted 1,4-benzenediole (hydroquinone) and substituted derivatives of hydroquinone in which the aryl hydrogens may be independently substituted by linear or branched, aliphatic or aromatic C₁-C₁₈ moiety. Hydroquinone-type compounds according to the present invention also include the corresponding methoxylated derivatives (hydroxyanisoles), like hydroquinone monomethylether or 2- and/or 3-tert-butyl-4-hydroxyanisole (butylated hydroxyanisole, BHA), which can also be further substituted as defined above. Preferred hydroquinone-type compounds are 4-methoxyphenol (para-methoxyphenol, MEHQ) and/or 2-methylhydrochinon (2-MDHQ; 2,5-Dihydroxytoluol) and/or 2- and/or 3-tert-butyl-4-hydroxyanisole.

In another preferred embodiment, the at least one stabilizer is selected from the group consisting of p-methoxyphenol, 4-tert-butylcatechol, tert-butylhydroquinone, 1,4-benzoquinone, 6-tert-butyl-2,4-xylenol, 2,6-di-tert-butyl-p-cresol, 2,6-di-tert-butylphenol, 1,1-diphenyl-2-picrylhydrazyl free radical, hydroquinone, nitroso benzene, 4-nitroso-3-methylphenol, sodium-1- naphthylamine-4-sulfonate and phenothiazine.

In another preferred embodiment, the at least one stabilizer is p-methoxyphenol.

In another preferred embodiment, in step d) the water solution that is saturated with oxygen and contains the at least one stabilizer, preferably p-methoxyphenol, is passed through the fixed-bed reactor at a flow rate in the range of ≥ 0.5 to ≤ 10 bed volume per hour. More preferably, in step d) the water solution is passed through the fixed-bed reactor at a flow rate in the range of ≥ 1 to ≤ 10 bed volume per hour.

In another preferred embodiment, in step d) the water solution that is saturated with oxygen and contains the at least one stabilizer is passed through the fixed-bed reactor in the direction of top to bottom or bottom to top. More preferably, in step d) the water solution that is saturated with oxygen and contains the at least one stabilizer is passed through the fixed-bed reactor in the direction of top to bottom.

In another preferred embodiment, in step d) humid air that is saturated with oxygen is passed through the fixed-bed reactor in the direction of bottom to top or bottom to top. More preferably, in step d) the water vapour that is saturated with oxygen is passed through the fixed-bed reactor in the direction of bottom to top.

In another preferred embodiment, the process further comprises the step of b) washing the fixed-bed reactor comprising activated carbon with water. In another preferred embodiment, the step b) is performed before step d). In another preferred embodiment, in step b) the water is passed through the fixed-bed reactor at a flow rate in the range of ≥ 0.5 to ≤ 10 bed volume per hour. More preferably, in step b) the water is passed through the fixed-bed reactor at a flow rate in the range of ≥ 1 to ≤ 10 bed volume per hour.

In another preferred embodiment, in step b) the washing of the fixed-bed comprising activated carbon with water is conducted in the direction of bottom to top or bottom to top. More preferably, in step b) the washing of the fixed-bed comprising activated carbon with water conducted in the direction of bottom to top.

In another preferred embodiment, the process comprises the step c) dumping the water from the fixed-bed reactor comprising activated carbon using air. In another preferred embodiment, step b) is performed before step d).

In another preferred embodiment, the step of c) is conducted in the direction of top to bottom or bottom to top. More preferably, the step c) is conducted in the direction of top to bottom.

In another preferred embodiment, the process further comprises the step of e) loading of the acrylamide solution to the activated fixed-bed reactor. In a preferred embodiment, the step e) is performed before step f). In step e), the acrylamide solution has a concentration in the range of ≥ 5.0 wt.-% to ≤ 30.0 wt.-%, based on the total weight of the solution. More preferably, the concentration is in the range of ≥ 10.0 wt.-% to ≤ 25.0 wt.-%, based on the total weight of the solution. Most preferably, the concentration is in the range of ≥ 10.0 wt.-% to ≤ 20.0 wt.-%, based on the total weight of the solution.

In another preferred embodiment, in step e) the loading of the solution comprising acrylamide is conducted in the direction of bottom to top or bottom to top. More preferably, in step e) the loading of the solution comprising acrylamide is conducted in the direction of bottom to top

In another preferred embodiment, in step f) the solution comprising acrylamide is passed through the fixed-bed reactor at a flow rate in the range of ≥ 0.1 to ≤ 10 bed volume per hour. More preferably, in step f) the solution comprising acrylamide is passed through the fixed-bed reactor at a flow rate in the range of ≥ 0.5 to ≤ 10 bed volume per hour. Even more preferably, in step f) the solution comprising acrylamide is passed through the fixed-bed reactor at a flow rate in the range of ≥ 1 to ≤ 10 bed volume per hour. Most preferably, in step f) the solution comprising acrylamide is passed through the fixed-bed reactor at a flow rate in the range of ≥ 1 to ≤ 5 bed volume per hour. In particular, in step f) the solution comprising acrylamide is passed through the fixed-bed reactor at a flow rate in the range of ≥ 1 to ≤ 5 bed volume per hour.

In another preferred embodiment, the fixed-bed reactor is maintained at a temperature in the range of ≥ 10 to ≤ 50 °C while performing all process steps a), d) and f), more preferably while performing all process steps a), b), c), d) e) and f). More preferably, the fixed-bed reactor is maintained at a temperature in the range of ≥ 15 to ≤ 50 °C. Even more preferably, the fixed-bed reactor is maintained at a temperature in the range of ≥ 15 to ≤ 40 °C. Most preferably, the fixed-bed reactor is maintained at a temperature in the range of ≥ 20 to ≤ 40 °C. In particular, the fixed-bed reactor is maintained at a temperature in the range of ≥ 20 to ≤ 30 °C.

In another preferred embodiment, the fixed-bed reactor is maintained at a temperature in the range of ≥ 15 to ≤ 30 °C while performing all process steps a), d) and f), more preferably while performing all process steps a), b), c), d) e) and f).

In another preferred embodiment, the fixed-bed reactor is maintained at a pressure in the range of ≥ 1 bar to ≤ 5 bar while performing all process steps a), d) and f), more preferably while performing all process steps a), b), c), d) e) and f). More preferably, the fixed-bed reactor is maintained at a pressure in the range of ≥ 1 bar to ≤ 3 bar. Most preferably the fixed-bed reactor is maintained at a pressure in the range of ≥ 1.5 bar to ≤ 2.5 bar. In particular, the fixed-bed reactor is maintained at a pressure in the range of ≥ 1 bar to ≤ 2 bar.

The presently claimed invention is associated with at least one of the following advantages:
- The content of the surface-active agents/defoamers comprising acrylamide are reduced to considerable quantity.
- The protein content in the solution comprising acrylamide is reduced to considerable quantity.
- The foaming behaviour of the solution comprising acrylamide is reduced in the downstream processes.
- The impure solution comprising acrylamide is purified using a continuous method.

In the following, there is provided a list of embodiments to further illustrate the present disclosure without intending to limit the disclosure to the specific embodiments listed below.

### Embodiments:

1. A process for the purification of a solution comprising acrylamide comprising at least the steps of:
   a) providing a fixed-bed reactor comprising activated carbon;
   d) treating the activated carbon with oxygen and at least one stabilizer; and
   f) passing the solution comprising acrylamide through the fixed-bed reactor to obtain a purified solution comprising acrylamide;
   wherein the pH of the solution comprising acrylamide is in the range of ≥ 6.0 to ≤ 9.
2. The process according to embodiment 1, wherein the solution comprising acrylamide has a pH in the range of ≥ 7 to ≤ 8.
3. The process according to embodiment 1 or 2, wherein the acrylamide is obtained by hydrolysis of acrylonitrile by a nitrile hydratase.
4. The process according to any of embodiments 1 to 3, wherein the acrylamide is present in the solution in a concentration in the range of ≥ 30.0 wt.-% to ≤ 60.0 wt.-%, based on the total weight of the solution.
5. The process according to any of embodiments 1 to 4, wherein the solution comprising acrylamide comprises at least one surface active agent.
6. The process according to embodiment 5, wherein the at least one surface active agent is present in an amount in the range of 1 ppm to 1000 ppm, based on the total weight of the solution.
7. The process according to any of embodiments 1 to 6, wherein the solution comprising acrylamide comprises at least one surface-active agent having a surface tension ≤ 60 m N/m
8. The process according to embodiments 5 to 7, wherein the at least one surface active agent is selected from the group consisting of polyalkylene glycol, alkyl polyalkylene glycol, alkyl polyalkoxyester, fatty acid polyglycol esters, polysiloxane glycol copolymers, silicone compounds and emulsions, oxalkylated compounds, mineral oil/synthetic blends, glycol/ester blends.
9. The process according to any of embodiments 1 to 8, wherein the activated carbon is selected from the group consisting of steam activated carbon, acid activated carbon or activated using at least one chemical activating agents selected from the group consisting of alkali metal hydroxides, alkali metal carbonates, alkali metal sulfide, alkali metal sulfates, alkaline earth metal carbonates, alkaline earth metal chlorides, alkaline earth metal sulfates and alkaline earth metal phosphates.
10. The process according to any of embodiments 1 to 9, wherein the activated carbon has a particle size in the range of ≥ 50 nm to ≤ 8 mm, determined according to sedigraph measurements.
11. The process according to any of embodiments 1 to 10, wherein the activated carbon has a surface area in the range of ≥ 300 to ≤ 2500 m²/g, determined according to BET surface area analysis.
12. The process according to any of embodiments 1 to 11, wherein the activated carbon has the porosity is in the range of ≥ 0.05 to ≤ 500 nm, determined according to sieve analysis.
13. The process according to any of embodiments 1 to 12, wherein the activated carbon is present in powdered, granular, pellet or cylindrical shape.
14. The process according to any of embodiments 1 to 12, wherein the bed volume of the activated carbon in the fixed-bed reactor is in the range of ≥ 10 to ≤ 80 %, based on the volume of the fixed-bed reactor.
15. The process according to any of embodiments 1 to 14, wherein in step d) the activated carbon is treated by using a water solution that is saturated with air and contains the at least one stabilizer
   or
   the activated carbon is treated with humid air and a water solution that contains the at least one stabilizer.
16. The process according to embodiment 14, wherein in step d) the water solution is passed through the fixed-bed reactor at a flow rate in the range of ≥ 0.1 to ≤ 10 bed volume per hour.
17. The process according to embodiment 15 or 16, wherein in step d) the water solution that is saturated with air and contains the at least one stabilizer is passed through the fixed-bed reactor in the direction of top to bottom or bottom to top.
18. The process according to embodiment 15, wherein in step d) the water vapour that is saturated with air is passed through the fixed-bed reactor in the direction of bottom to top or bottom to top.
19. The process according to any of embodiments 1 to 18, wherein the at least one stabilizer is selected from the group consisting of p-methoxyphenol, 4-tert-butylcatechol, tert-butylhydroquinone, 1,4-benzoquinone, 6-tert-butyl-2,4-xylenol, 2,6-di-tert-butyl-p-cresol, 2,6-di-tert-butylphenol, 1,1-diphenyl-2-picrylhydrazyl free radical, hydroquinone, nitroso benzene, 4-nitroso-3-methylphenol, sodium-1- naphthylamine-4-sulfonate and phenothiazine.
20. The process according to embodiment 19, wherein the at least one stabilizer is p-methoxyphenol.
21. The process according to any of embodiments 1 to 20 further comprising the step of b) washing the fixed-bed reactor comprising activated carbon with water before step d).
22. The process according to embodiment 21, wherein in step b) the washing of the fixed-bed comprising activated carbon with water conducted in the direction of bottom to top or bottom to top.
23. The process according to any of embodiments 1 to 22 further comprising the step of e) loading of the acrylamide solution to the activated fixed-bed reactor before step f).
24. The process according to embodiment 23, wherein in step e) the loading of the solution comprising acrylamide is conducted in the direction of bottom to top or bottom to top.
25. The process according to embodiment 1, wherein in step f) the solution comprising acrylamide is passed through the fixed-bed reactor at a flow rate in the range of ≥ 0.1 to ≤ 10 bed volume per hour.
26. The process according any of embodiments 1 to 25, wherein the fixed-bed reactor is maintained at a temperature in the range of ≥ 10 to ≤ 50 °C.
27. The process according to embodiment 26, wherein the fixed-bed reactor is maintained at a temperature in the range of ≥ 15 to ≤ 30 °C.
28. The process according any of embodiments 1 to 27, wherein the fixed-bed reactor is maintained at a pressure in the range of ≥ 1 bar to ≤ 5 bar.
29. The process according to any of embodiments 1 to 28, wherein the purified solution comprises at least one surface-active agent in an amount in the range of ≥ 0.001 ppm to ≤ 3 ppm, based on the total weight of the solution.
30. A process for purification of a solution comprising acrylamide comprising at least the steps of:
   a) providing a fixed-bed reactor comprising activated carbon;
   b) washing the fixed-bed reactor comprising activated carbon with water;
   c) dumping the water using air;
   d) treating the activated carbon with oxygen and at least one stabilizer;
   e) loading of the acrylamide solution to the fixed-bed reactor comprising activated carbon; and
   f) passing the solution comprising acrylamide through the fixed-bed reactor to obtain a purified solution comprising acrylamide.
31. The process according to embodiment 30, wherein in step c) the dumping of the water from the fixed-bed reactor comprising activated carbon is conducted in the direction of top to bottom or bottom to top.
32. The process according to any of embodiments 30 to 31, wherein the fixed-bed reactor is maintained at a temperature in the range of ≥ 10 to ≤ 50 °C.
33. The process according to embodiment 32, wherein the fixed-bed reactor is maintained at a temperature in the range of ≥ 15 to ≤ 30 °C.
34. The process according to any of embodiments 30 to 31, wherein the fixed-bed reactor is maintained at a pressure in the range of ≥ 1 bar to ≤ 5 bar.

While the presently claimed invention has been described in terms of its specific embodiments, certain modifications and equivalents will be apparent to those skilled in the art and are intended to be included within the scope of the presently claimed invention

### Examples

The presently claimed invention is illustrated in detail by non-restrictive working examples which follow. More particularly, the test methods specified hereinafter are part of the general disclosure of the application and are not restricted to the specific working examples.

### Materials:

Bioacrylamide solution (BioACM) is available from BASF and Sigma Aldrich.
p-methoxyphenol (MeHQ) is available from Sigma Aldrich.
Chemviron® CAL I 12x40 (steam activated carbon with BET = 1050 m²/g, Part. size = 0.9-1.1 mm)
Chemviron® CPG LF 12x40 (steam activated acid washed carbon with BET = 950 m²/g, Part. size = 1.2-1.4 mm)
Chemviron® Aquacarb 207C 12x30 (coconut based carbon with BET = 1100 m²/g, Part. size = 1.2 mm)
Norit® (Cabot) C gran (phosphoric acid activated with BET Surface = 1100 m²/g, Part. size 0.42-1.70 mm).

### Reactor:

The fixed bed carbon column was built with the following dimensions:
- Height: 13 cm
- Diameter: 1.6 cm
- Internal Volume: 26 mL
- Carbon loading: 10 g

### Methods:

Method for measuring the forming behaviour: A 100 mL plastic measuring cylinder was filled with 50 mL of acrylamide, then air was bubbled through a frit at a constant flow rate of 6 L/h (gassing) until the foam reached the 100 mL level, at that point the frit was removed, and the foam was allowed to rest. Different measurements were taken:
- Maximum rise in cylinder (in mL)
- Time for the froth to reach 100 mL (in s)
- Time for the froth to collapse (in s)

### Method for measuring the protein content:

The protein content was measured by determining the total amino acid content and the amino acid composition. The liquid samples were first purified by solid-phase extraction (SPE). The eluate was concentrated and hydrolyzed with hydrochloric acid. The amino acid tryptophan was lost through hydrolysis and methionine was lost partially. Asparagine and glutamine were deamidated by the acid hydrolysis into their corresponding acids, therefore, the sum of both amino acids (GLX and ASX) is given in the results. The results refer to 1 mL each of the starting sample and were recalculated accordingly.

### Sample preparation:

1 mL of the solution comprising acrylamide was purified by SPE and the eluate was concentrated. The concentrate was dissolved in 1 ml of 6 M HCI and transferred to three 0.3 mL each into hydrolysis vessels. The samples were hydrolyzed overnight at 100 ° C and concentrated. The concentrate was then taken up in 30 *µ*L of water and shaken. The amino acids were derivatized and analyzed

### HPLC method:

• Eluent A: ACCQ-Tag Ultra Eluent A
• Eluent B: ACCQ-Tag Ultra Eluent B
• Column: ACCQ-Tag Ultra Column
• Flow: 0.5 mL/min
• Temp.: 43 ° C
• Gradient:

| | |
|---|---|
| 0 min | 1% B |
| 0.28 min | 1% B |
| 2.8 min | 2% B |
| 4.2 min | 4% B |
| 5.6 min | 7% B |
| 6.3 min | 12% B |
| 8.4 min | 14% B |
| 9.1 min | 22% B |
| 10.5 min | 24% B |
| 10.6 min | 90% B |
| 11.3 min | 90% B |

• Detection: UV; 260 nm
Defoamer/ surface active agents quantification
Deformer/ surface active agent used is Pluriol® which is commercially available form BASF. The samples were quantified using LCMS. The solution comprising acrylamide were injected directly in the LC/MS
Mobile phase A: water; 0.1% HCOOH
Mobile phase B: acetonitrile; 0.1% HCOOH
Column: Kinetex; C18; 100x2.1 mm; 1.7 *µ*m (Ph080)
Flow: 0.6 mL/min
Temp.: 40 °C
Gradient:

| | |
|---|---|
| 0 min | 20% B |
| 1.5 min | 20% B |
| 2 min | 99% B |
| 9 min | 99% B |
| 9.1 min | 20% B |
| 11 min | 20% B |

Detection: MS-ESI SingleQuad; pos. Sim mode (512.80; 532.15; 551.55; 570.90; 590.25; 609.60; 628.95; 648.35; 667.70; 687.05; 706.40; 725.75; 745.10; 764.45; 783.85; 803.20; 822.55; 841.90 m /z)
Evaluation via "SIM" (628.95; 648.35; 667.70; 687.05; 706.40; 725.75 m/z).

### Purification process

Reference examples (without using fixed bed column) showing purification of solution comprising acrylamide in batch mode. The solution comprising acrylamide was stirred for 24 hours with different quantity of activated carbon. The treated solution comprising acrylamide was filtered and 50 mL was transferred to a 100 mL measuring cylinder. Air or nitrogen was bubbled through the 50 mL solution in the measuring cylinder and the time for the froth to reach 100 mL height as well and time to collapse the froth to original level were measured. The results are tabulated in table 1.

**Table 1:**

| Sample | Activated carbon concentration (%) | MeHQ conc. (ppm) | Maximum rise in 100 mL cylinder (mL) | Time for froth to reach 100 ml (s) | Time for collapse of froth (s) |
|---|---|---|---|---|---|
| reference | - | 15 | 100 | 23 | 148 |
| Chemviron® CAL I | 1 | n.m. | 77 | - | - |
| Chemviron® CPG LF | 1 | n.m. | 79 | - | - |
| Chemviron® Aquacarb 207C | 1 | n.m. | 100 | 31 | n.m. |
| Norit® (Cabot) C gran | 1 | n.m. | 80 | - | - |
| Chemviron® CAL I | 0.1 | 13 | 100 | 32 | 73 |
| Chemviron® CPG LF | 0.1 | 14 | 100 | 26 | 80 |
| Chemviron® Aquacarb 207C | 0.1 | 14 | 100 | 26 | 147 |
| Norit® (Cabot) C gran | 0.1 | 13 | 100 | 26 | 61 |

| | | | | | |
|---|---|---|---|---|---|
| n.m.- not measured. | | | | | |

### Fixed bed activated carbon column:

The column was filled with 10 g of activated carbon Cabot Norit® GAC (Mesh 1240, 0.42-1.70 mm, BET Surface = 1100 m²/g) and saturated with oxygen and 1000 ppm of p-methoxyphenol (MeHQ). The solution comprising acrylamide (containing 10 ppm Pluriol® and bovine serum albumin as surface active agent) was pumped at a constant flow of bed volume per hour (26 mL/h = 1 BV/h). The quantity of the Pluriol® and the protein in the purified sample were measured and result is tabulated in table 2.

A peristaltic pump (Pharmacia P1) was used to pump the acrylamide solution through the column at a constant flow rate of 26 mL/h (1 BV/h).

**Table 2: Fix bed activated carbon column using Cabot Norit carbon**

| Sample | Transmittance (%) | MeHQ conc. (ppm) | Maximum rise in cylinder (mL) | Time for froth to reach 100 ml (s) | Time for collapse of froth (s) | Pluriol® conc. (ppm) | Surface Tension (mS/m) | Protein content (mg/L) |
|---|---|---|---|---|---|---|---|---|
| Starting solution | 98.8 | 15 | 100 | 20 | 165 | 8.5 | 49.3 | 12 |
| After 500 mL | 98.8 | 11 | 80 | - | - | <0.1 | 66.3 | 5 |
| After 5 L | 99.6 | 10 | 100 | 24 | 16 | <0.1 | n.m. | n.m. |
| After 10 L | 98.9 | 10 | 100 | 21 | 20 | 0.1 | 66.1 | 7 |
| After 20 L | n.m. | n.m. | 100 | 21 | 19 | 0.5 | n.m. | n.m. |
| After 30 L | n.m. | 12 | 100 | 22 | 30 | 1.8 | 65.1 | 5 |
| After 40 L* | n.m. | 12 | 70 | - | - | 2.1 | n.m. | n.m. |
| After 50 L* | n.m. | 13 | 72 | - | - | 2.5 | n.m. | n.m. |

| | | | | | | | | |
|---|---|---|---|---|---|---|---|---|
| n.m.- not measured. | | | | | | | | |

The purification using above column was continued for 2 months and no polymerization of the acrylamide was observed even after continuous purification of 50 L solution (almost 2000 BV of acrylamide solution).

The similar experiment was repeated without saturating the fixed bed activated carbon column with p-methoxyphenol (MeHQ). However, after 200 hours the column was clogged due to polymerization of the acrylamide monomer.

In another experiment, the solution comprising acrylamide was passed through a fixed bed activated carbon without saturating the column with oxygen. Although, the column was saturated with p-methoxyphenol (MeHQ) did not stop the polymerization of the acrylamide monomer.

It is evident from above experiments, that the fixed bed activated carbon column saturated with oxygen and at least one stabilizer provides efficient mechanism for purification of a solution comprising acrylamide monomer without causing polymerization. It also effectively reduces the impurities which causes the forming behaviour.

## Claims

1. A process for the purification of a solution comprising acrylamide comprising at least the steps of:
a) providing a fixed-bed reactor comprising activated carbon;
d) treating the activated carbon with oxygen and at least one stabilizer; and
f) passing the solution comprising acrylamide through the fixed-bed reactor to obtain a purified solution comprising acrylamide;
wherein the pH of the solution comprising acrylamide is in the range of ≥ 6.0 to ≤ 9.

2. The process according to embodiment 1, wherein the solution comprising acrylamide has a pH in the range of ≥ 7 to ≤ 8.

3. The process according to any of claims 1 to 2, wherein the acrylamide is present in the solution in a concentration in the range of ≥ 30.0 wt.-% to ≤ 60.0 wt.-%, based on the total weight of the solution.

4. The process according to any of claims 1 to 3, wherein the solution comprising acrylamide comprises at least one surface active agent.

5. The process according to claim 4, wherein the at least one surface active agent is present in an amount in the range of ≥ 1 ppm to ≤ 1000 ppm, based on the total weight of the solution.

6. The process according to any of claims 1 to 5, wherein in step d) the activated carbon is treated by using a water solution that is saturated with air and contains the at least one stabilizer
or
the activated carbon is treated with humid air and a water solution that contains the at least stabilizer.

7. The process according to claim 6, wherein in step d) the water solution is passed through the fixed-bed reactor at a flow rate in the range of ≥ 0.5 to ≤ 10 bed volume per hour.

8. The process according to claim 6 or 7, wherein in step d) the water solution that is saturated with air and contains the at least one stabilizer is passed through the fixed-bed reactor in the direction of top to bottom.

9. The process according to any of claims 1 to 8, wherein the at least one stabilizer is selected from the group consisting of p-methoxyphenol, 4-tert-butylcatechol, tert-butylhydroquinone, 1,4-benzoquinone, 6-tert-butyl-2,4-xylenol, 2,6-di-tert-butyl-p-cresol, 2,6-di-tert-butylphenol, 1,1-diphenyl-2-picrylhydrazyl free radical, hydroquinone, nitroso benzene, 4-nitroso-3-methylphenol, sodium-1-naphthylamine-4-sulfonate and phenothiazine.

10. The process according to any of claims 1 to 9 further comprising the step of b) washing the fixed-bed reactor comprising activated carbon with water before step d).

11. The process according to any of claims 1 to 10 further comprising the step of e) loading of the acrylamide solution to the activated fixed-bed reactor before step f).

12. The process according to claim 1, wherein in step f) the solution comprising acrylamide is passed through the fixed-bed reactor at a flow rate in the range of ≥ 0.5 to ≤ 10 bed volume per hour.

13. The process according any of claims 1 to 12, wherein the fixed-bed reactor is maintained at a temperature in the range of ≥ 10 to ≤ 50 °C.

14. A process for purification of a solution comprising acrylamide comprising at least the steps of:
a) providing a fixed-bed reactor comprising activated carbon;
b) washing the fixed-bed reactor comprising activated carbon with water;
c) dumping the water using air;
d) treating the activated carbon with oxygen and at least one stabilizer;
e) loading of the acrylamide solution to the fixed-bed reactor comprising activated carbon; and
f) passing the solution comprising acrylamide through the fixed-bed reactor to obtain a purified solution comprising acrylamide.

15. The process according to claim 14, wherein the fixed-bed reactor is maintained at a temperature in the range of ≥ 10 to ≤ 50 °C.
